# FASCICULE DE BREVET EUROPEEN

(11) **EP 2 419 783 B1**
(45) Date de publication et mention de la délivrance du brevet: **20.03.2013**
(21) Numéro de dépôt: 10717192.8
(22) Date de dépôt: 15.04.2010
(51) Int. Cl.: G02C 13/00

(54) **PROCÉDÉ DE DÉTERMINATION D'AU MOINS UN PARAMÈTRE GÉOMÉTRICO-POSTURAL D'IMPLANTATION D'UNE MONTURE DE LUNETTES DE CORRECTION VISUELLE SUR LE VISAGE D'UN PORTEUR DANS SA POSTURE ANATOMIQUE**
VERFAHREN ZUR BESTIMMUNG VON MINDESTENS EINEM GEOMETRISCHEM HALTUNGSPARAMETER BEI DER ANPASSUNG EINER KORRIGIERENDEN BRILLENFASSUNG AN DAS GESICHT EINES TRÄGERS IN SEINER ANATOMISCHEN HALTUNG
METHOD FOR DETERMINING AT LEAST ONE GEOMETRIC POSTURAL PARAMETER WHEN FITTING A CORRECTIVE SPECTACLE FRAME ON THE FACE OF A WEARER IN THE ANATOMICAL POSTURE THEREOF

(30) Priorité: 17.04.2009 FR 0901887
(43) Date de publication de la demande: 22.02.2012
(73) Titulaire: Essilor International (Compagnie Générale d'Optique), 94220 Charenton-le-Pont (FR)
(72) Inventeur: CHAUVEAU, Jean-Pierre, F-94220 Charenton Le Pont (FR); BARANTON, Konogan, F-94220 Charenton Le Pont (FR); PEDRONO, Claude, F-94220 Charenton Le Pont (FR)
(74) Mandataire: Chauvin, Vincent
(86) Numéro de dépôt international: PCT/FR2010/000310
(87) Numéro de publication internationale: WO 2010/119190

(56) Documents cités:
- WO-A-2009/024681
- FR-A- 2 860 887
- FR-A- 2 906 047
- FR-A- 2 914 173
- FR-A- 2 915 290

## Description

### DOMAINE TECHNIQUE AUQUEL SE RAPPORTE L'INVENTION

La présente invention concerne un procédé de détermination d'au moins un paramètre géométrico-postural d'implantation d'une monture de lunettes de correction visuelle sur le visage d'un porteur dans sa posture anatomique.

### ARRIÈRE-PLAN TECHNOLOGIQUE

Dans le cadre du montage de lentilles ophtalmiques de correction visuelle dans une monture de lunettes, on cherche actuellement à prendre mieux en compte des paramètres géométrico-posturaux individuels, dits de conception optique personnalisée, attachés au porteur et à la monture qu'il a choisie.

Les paramètres géométrico-posturaux recherchés sont liés à la fois à la géométrie de la tête du porteur et de la monture de lunettes choisie, ainsi qu'à la posture du porteur. Ils comprennent notamment la configuration spatiale de chaque lentille par rapport à la tête du porteur, dans les conditions du porté. Cette configuration spatiale est notamment déterminée par l'angle pantoscopique de chaque lentille au porté (angle que forme le plan général de la lentille par rapport à la verticale) et la hauteur des yeux du porteur par rapport au bord inférieur de la lentille.

Pour déterminer cette configuration spatiale, l'opticien place une paire de lunettes de présentation sur le nez du porteur. Les lunettes de présentation comportent la monture choisie par le porteur et des lentilles non correctrices montées dans les cercles de la monture.

La mesure de la hauteur des yeux du porteur par rapport au bord inférieur de la monture peut être effectuée manuellement : l'opticien observe le porteur de face et réalise une mesure à l'estime, au moyen d'un réglet, de la distance entre la pupille d'un oeil et le bord inférieur de la lentille de présentation.

On a proposé d'automatiser la détermination de ces paramètres géométrico-posturaux en la réalisant à partir d'une ou plusieurs captures d'images du porteur équipé de la monture de lunettes choisie. Ces images sont traitées pour déterminer les paramètres recherchés.

Par exemple, FR 2 915 290 décrit un procédé de détermination d'au moins un paramètre géométrico-postural d'implantation d'une monture de lunettes de correction visuelle sur le visage d'un porteur dans sa posture anatomique, selon lequel on réalise les étapes suivantes :
a) on définit, sur la tête du porteur et/ou sur la monture de lunettes équipant le porteur, des moyens de repérage pour repérer :
   - la position d'un référentiel lié à la tête du porteur dans un référentiel lié à un dispositif de capture d'images et
   - l'inclinaison de ce référentiel lié à la tête du porteur dans le plan sagittal de la tête du porteur, autour d'un centre de rotation de la tête du porteur,
b) on capture au moins une image de la tête dudit porteur par le dispositif de capture d'images
c) on identifie, sur ladite au moins une image capturée, l'image d'au moins un premier point anatomique remarquable du visage du porteur,
d) on détermine le paramètre géométrico-postural recherché.

Cependant, qu'elles soient manuelles ou automatisées, ces méthodes de détermination des paramètres géométrico-posturaux du porteur souffrent d'une grande imprécision. En effet, pour effectuer une mesure précise au réglet ou par traitement des images capturées, il faut que la tête du porteur soit dans sa posture anatomique au moment de la mesure ou de la capture d'images. Cette posture anatomique est aussi appelée position orthostatique.

Or, pour des raisons de rapidité d'exécution et de confort de l'opticien comme du porteur, la position de la tête du porteur lors de la prise d'image n'est pas contrôlée. Or, une déviation de 1 degré de l'inclinaison de la tête dans son plan sagittal par rapport à la posture anatomique introduit une erreur de 1 degré sur la mesure de l'angle pantoscopique et une erreur de 0,5 millimètre sur la mesure des hauteurs des yeux.

### OBJET DE L'INVENTION

Afin de remédier aux inconvénients de l'état de la technique, on propose selon l'invention un procédé de détermination des paramètres géométrico-posturaux du porteur assurant à l'opticien que ces paramètres sont déterminés dans la posture anatomique du porteur.

A cet effet, on propose selon l'invention un procédé de détermination d'au moins un paramètre géométrico-postural d'implantation d'une monture de lunettes de correction visuelle sur le visage d'un porteur dans sa posture anatomique, selon lequel on réalise les étapes suivantes :
a) on définit, sur la tête du porteur et/ou sur la monture de lunettes équipant le porteur, des moyens de repérage pour repérer :
   - la position d'un référentiel lié à la tête du porteur dans un référentiel lié à un dispositif de capture d'images et
   - l'inclinaison de ce référentiel lié à la tête du porteur dans le plan sagittal de la tête du porteur autour d'un centre de rotation de la tête du porteur,
b) on capture une série d'au moins deux images de la tête dudit porteur par le dispositif de capture d'images,
c) on déterminé au moins une position, dans le référentiel lié au dispositif de capture d'images, du centre de rotation de la tête du porteur dans le plan sagittal,
d) on identifie, sur chacune desdites au moins deux images capturées, l'image d'au moins un premier point anatomique remarquable du visage du porteur,
e) on détermine, pour chacune desdites au moins deux images capturées, l'angle d'inclinaison d'un plan anatomique de la tête du porteur passant par ledit centre de rotation et par ledit au moins un premier point anatomique remarquable et perpendiculaire audit plan sagittal, par rapport à un plan de référence connu dans le référentiel lié au dispositif de capture d'images,
f) on compare cet angle d'inclinaison avec une valeur prédéterminée,
g) on détermine le paramètre géométrico-postural recherché en fonction du résultat de cette comparaison.

Le plan anatomique choisi est un plan dont l'angle d'inclinaison dans la posture anatomique est connu.

La posture anatomique normale de la tête du porteur correspond à la position de la tête du porteur dans l'espace pour laquelle le plan de Francfort de la tête du porteur s'étend selon un plan horizontal. On dit également que le porteur est alors dans une position orthostatique, position dans laquelle il réalise un minimum d'efforts.

Le plan de Francfort est défini comme le plan passant par les points orbitaires inférieurs OR et le porion PO du porteur, le porion étant le point du crâne le plus élevé du conduit auditif, qui correspond au tragion TR de l'oreille, c'est-à-dire le point le plus élevé du tragus de l'oreille.

Ainsi, en choisissant un plan anatomique dont l'orientation par rapport au plan de Francfort est proche d'une valeur connue déterminée statistiquement pour une population d'individu donnée, l'orientation de ce plan anatomique dans la posture anatomique du porteur est déterminée facilement.

Grâce à ce procédé, on détermine sur quelles images de la série d'images capturées la tête du porteur est la plus proche de sa posture anatomique afin de déterminer les paramètres géométrico-posturaux du porteur directement à partir d'une image proche de la posture anatomique ou afin de les calculer dans la posture anatomique.

La précision des paramètres géométrico-posturaux ainsi déterminés est accrue ce qui autorise une adaptation plus précise des lentilles de correction ophtalmique à la monture choisie et optimise le confort du porteur.

Selon une première caractéristique avantageuse du procédé selon l'invention, à l'étape g),
- on sélectionne l'image de ladite série d'images capturées pour laquelle l'écart entre l'angle d'inclinaison déterminé à l'étape e) et ladite valeur prédéterminée est le plus petit,
- on détermine le paramètre géométrico-postural recherché par traitement de cette image sélectionnée.

La détermination des paramètres est ainsi rapide et aisée, sans étape comportant des calculs additionnels.

Selon une autre caractéristique avantageuse du procédé selon l'invention, à l'étape g),
- on corrige le paramètre géométrico-postural calculé en fonction de l'écart entre l'angle d'inclinaison déterminé à l'étape e) pour ladite image sélectionnée et ladite valeur prédéterminée.

Selon une autre caractéristique avantageuse du procédé selon l'invention, à l'étape g),
- on sélectionne les deux images de ladite série d'images capturées pour lesquelles l'écart entre l'angle d'inclinaison déterminé à l'étape e) et ladite valeur prédéterminée est le plus petit,
- on calcule, à partir de ces deux images sélectionnées, une modélisation d'au moins une partie du visage du porteur correspondant à l'image qui serait effectivement capturée si la valeur de l'angle d'inclinaison dudit plan anatomique était égale à ladite valeur prédéterminée,
- on détermine, à partir de cette modélisation, le paramètre géométrico-postural recherché.

Les paramètres géométrico-posturaux sont ainsi déterminés très précisément.

Selon une autre caractéristique avantageuse du procédé selon l'invention, à l'étape a), lesdits moyens de repérage comportent un dispositif de repérage monté directement sur la tête du porteur ou sur la monture de lunettes de ce porteur. Ces moyens de repérage peuvent également comporter une mesure en trois dimensions, sans contact, de la tête du porteur sur laquelle est positionnée la monture choisie.

Ce dispositif de repérage se présente par exemple sous la forme d'un accessoire qui peut être aisément clippé sur la monture de lunettes du porteur ou monté sur sa tête. A partir de l'image d'un tel dispositif, sa position et son orientation par rapport au dispositif de capture d'images peuvent être entièrement déterminées.

En variante, lesdits moyens de repérage comportent des marques de repère disposées sur la tête du porteur.

Selon une autre caractéristique avantageuse du procédé selon l'invention, à l'étape d), on identifie, sur chacune desdites au moins deux images capturées, l'image d'au moins une commissure nasale ou temporale d'un oeil du porteur et, à l'étape e), on détermine l'angle d'inclinaison du plan anatomique de la tête du porteur passant par cette commissure et par le centre de rotation correspondant à cette image.

Selon une autre caractéristique avantageuse du procédé selon l'invention, à l'étape d), on identifie sur chacune desdites au moins deux images capturées, la position du point le plus bas du nez du porteur, appelé point sous nasal, et, à l'étape e), on détermine l'angle d'inclinaison du plan anatomique de la tête du porteur passant par ce point sous nasal et le centre de rotation correspondant à cette image.

Selon une autre caractéristique avantageuse du procédé selon l'invention, à l'étape g), le paramètre géométrico-postural déterminé comprend l'une au moins des grandeurs suivantes :
- l'angle pantoscopique de la monture équipant le porteur,
- la hauteur des centres de rotation des yeux du porteur par rapport au bord inférieur de cette monture.

Selon une autre caractéristique avantageuse du procédé selon l'invention, à l'étape b), le porteur effectuant un mouvement de hochement vertical de la tête sensiblement dans le plan sagittal, on capture une série d'au moins deux images de la tête du porteur par le dispositif de capture d'images, pour au moins deux inclinaisons différentes de la tête.

Avantageusement alors, à l'étape c), on détermine la position du centre de rotation (Ai) à partir des positions et des inclinaisons du repère lié à la tête du porteur fournies par les moyens de repérage et correspondant à au moins deux desdites images capturées.

En outre, à l'étape c), la détermination du centre de rotation est réalisée à partir des positions et des inclinaisons du repère lié à la tête du porteur correspondant à un groupe d'images capturées successivement de ladite série d'images.

Selon une première variante du procédé selon l'invention, à l'étape b), toutes les images capturées du porteur sont des images sensiblement frontales du porteur, c'est-à-dire capturées dans un plan sensiblement perpendiculaire au plan sagittal de la tête du porteur.

Selon une deuxième variante du procédé selon l'invention,
- à l'étape b), on capture au moins une image sensiblement de profil de la tête du porteur, c'est-à-dire capturée dans un plan sensiblement parallèle au plan sagittal de la tête du porteur,
- à l'étape c), la détermination de la position dudit centre de rotation de la tête du porteur comprend l'identification, sur ladite image sensiblement de profil capturée à l'étape b), d'un deuxième point anatomique remarquable.

Avantageusement alors, à l'étape c), l'identification dudit deuxième point anatomique remarquable est réalisée :
- par traitement de ladite image sensiblement de profil capturée à l'étape b) et reconnaissance sur cette image d'une forme associée à ce deuxième point anatomique remarquable ou
- par l'affichage sur un écran de ladite image sensiblement de profil capturée à l'étape b) et la mise en oeuvre de moyen de pointage dudit deuxième point anatomique remarquable.

### DESCRIPTION DÉTAILLÉE D'UN EXEMPLE DE RÉALISATION

La description qui va suivre, en regard des dessins annexés, donnée à titre d'exemple non limitatif, fera bien comprendre en quoi consiste l'invention et comment elle peut être réalisée.

Sur les dessins annexés :
- la figure 1 est une vue en perspective du dispositif de repérage de la figure 2 fixé sur la paire de lunettes de présentation et un dispositif de capture d'images qui communiquent avec un système de traitement et de calcul ;
- la figure 2 est une vue de face de la tête d'un porteur équipé des lunettes de présentation dans sa posture anatomique et d'un dispositif de repérage dans sa posture anatomique ;
- la figure 3 est une vue de profil de la tête d'un porteur équipé des lunettes de présentation et d'un dispositif de repérage dans sa posture anatomique ;
- la figure 4 est une vue en perpective de la tête d'un porteur dans sa posture anatomique ;
- la figure 5 est une vue de face du dispositif de repérage de la figure 2 ;
- la figure 6 est une vue schématique relative à l'étape de détermination de l'axe horizontal de rotation instantané de la tête du porteur ;
- la figure 7 est une vue schématique relative à l'étape de détermination de l'angle d'inclinaison d'un plan anatomique par rapport au plan de référence lié au dispositif de capture d'images,
- la figure 8 est une vue schématique d'un oeil du porteur et de la monture, dans un plan parallèle au plan sagittal et passant par l'oeil considéré, illustrant une étape de correction d'un paramètre géométrico-postural déterminé.

Dans la description qui suit, l'opticien détermine des paramètres géométrico-posturaux du porteur équipé d'une monture 10 de lunettes dans la configuration du porté, le porteur assumant sa posture anatomique.

Ces paramètres sont ensuite utilisés pour le montage des lentilles ophtalmiques dans la monture.

Le porteur est dans une configuration assise ou debout.

Le plan facial, appelé plan vertical oeil PVO, est le plan perpendiculaire au plan de Francfort passant par les centres de rotation CROG, CROD des yeux.

Le plan sagittal PSAG, ou plan médian vertical de la tête du porteur est le plan orthogonal au plan vertical oeil PVO et au plan de Francfort PF, passant par le milieu des deux centres de rotation CROD, CROG des yeux OD, OG.

La médiatrice AO des yeux OD, OG correspond à l'intersection du plan sagittal PSAG et du plan de Francfort PF.

Le plan de Camper est le plan qui passe par les deux porions et le point le plus bas du nez, appelé le point sous nasal.

Le plan neuro-orbital PNO est le plan qui passe par les deux porions et les endocanthions, c'est-à-dire les commissures des yeux situées du côté nasal de chaque oeil.

Les paramètres géométrico-posturaux recherchés sont obtenus ici à partir de captures d'images du porteur équipé de la monture 10 de lunettes choisie. Cette monture 10 accueille des lentilles de présentation 100, 101 non correctrices qui équipent, pour la vente et les prises de mesure, la monture et qui se substituent, pour la détermination de paramètres géométrico-posturaux relatifs au porteur et à la monture, à la lentille correctrice à concevoir.

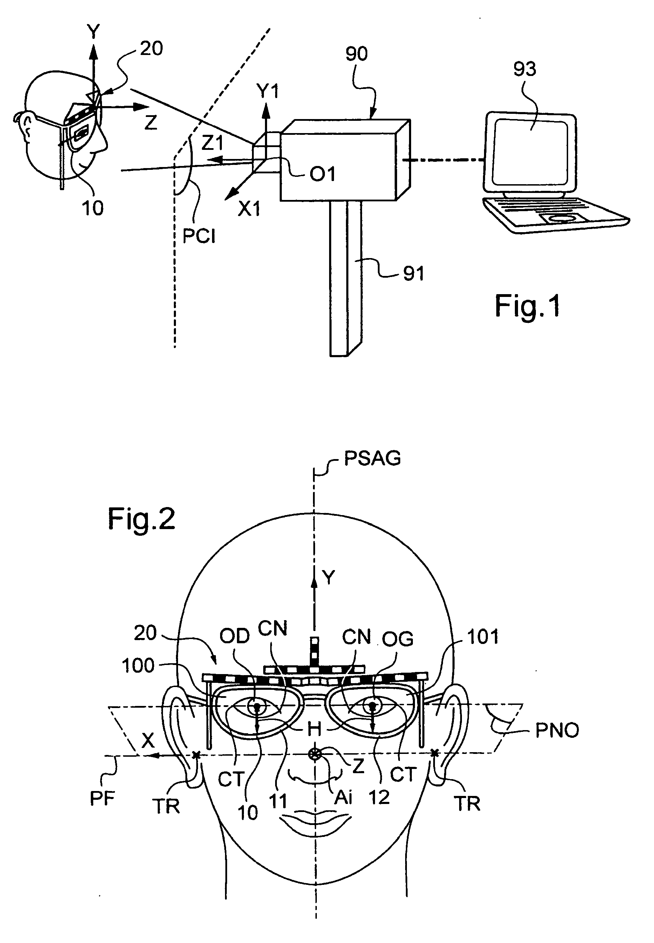

La paire de lunettes de présentation comporte la monture 10 choisie par le porteur et des lentilles de présentation droite et gauche 100, 101 (non correctrices). Dans l'exemple illustré sur la figure 2, la paire de lunettes est de type cerclée, c'est-à-dire que les lentilles sont montées dans les cercles 11, 12 de la monture 10.

En variante, la paire de lunettes de présentation peut être de type percée, c'est-à-dire que les lentilles sont percées et maintenues chacune par une extrémité du pontet nasal et une extrémité de la branche associée à la lentille, qui coopèrent avec des trous de perçage.

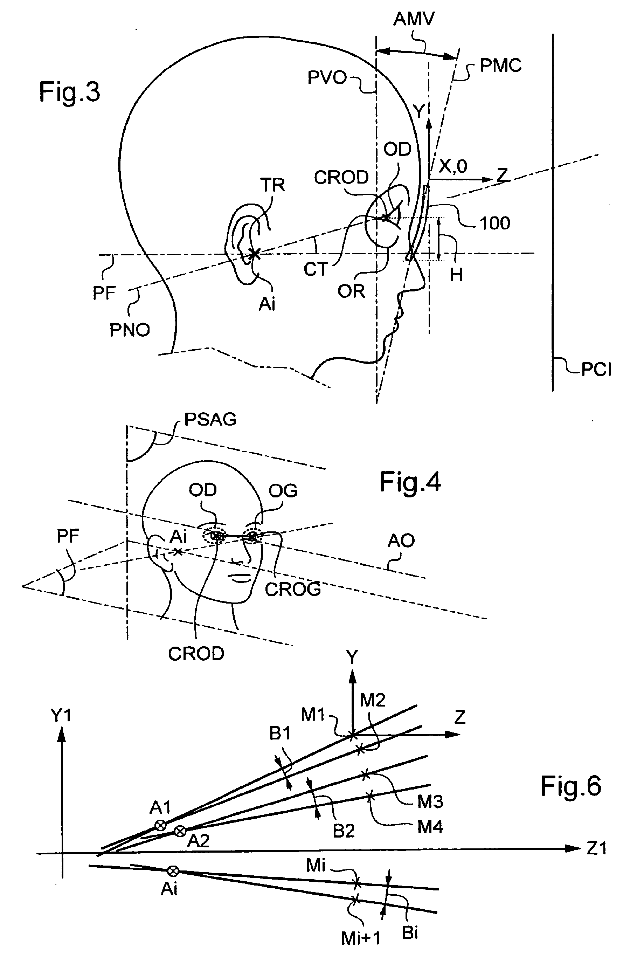

Les paramètres géométrico-posturaux recherchés correspondent par exemple à :
- l'orientation de chaque lentille correctrice à monter sur la monture par rapport à la tête du porteur et notamment l'angle pantoscopique AMV (figure 3) entre le plan moyen PMC des deux lentilles 100, 101 (ou encore des deux cercles 11, 12 de la monture 10) et le plan vertical oeil PVO,
- la distance H (figure 2), dans la configuration du porté, entre le bord inférieur de la lentille et la pupille ou le centre de rotation de l'oeil correspondant du porteur suivant une direction verticale parallèle au plan sagittal PSAG, appelée hauteur des yeux H.

Cette hauteur des yeux H correspond également à la distance entre la face interne du cercle 11, 12 de la monture 10 et la pupille ou le centre de rotation de l'oeil OD, OG correspondant du porteur suivant cette direction dans le cas d'une monture de lunettes cerclée, comme représenté dans l'exemple de la figure 2.

### Dispositif

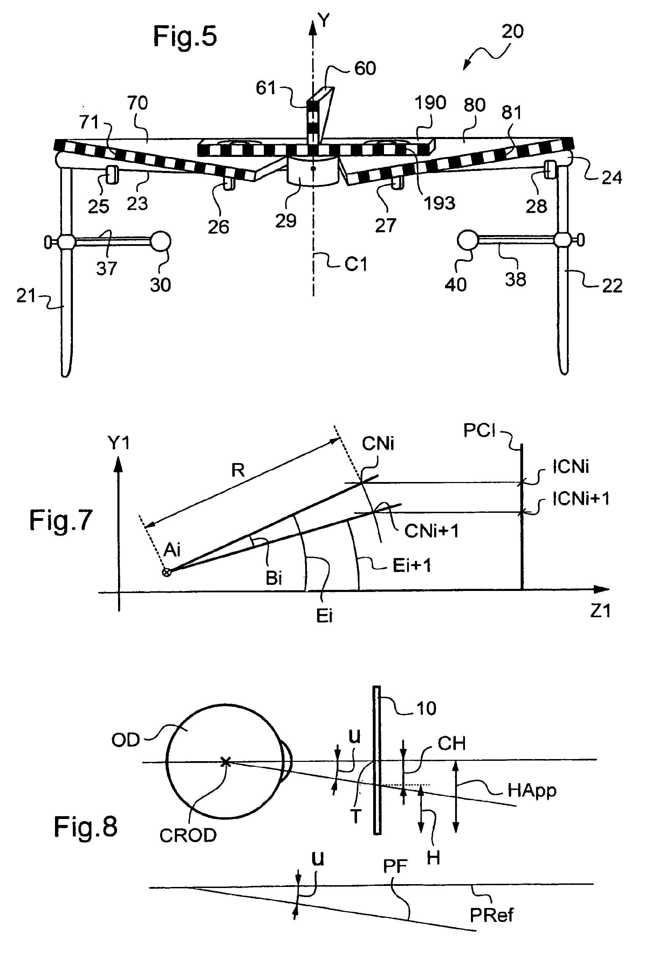

Aux figures 1 et 5, on a représenté un dispositif de détermination de paramètres géométrico-posturaux individuels d'un porteur équipé d'une paire de lunettes de présentation.

Ce dispositif comporte des moyens de repérage de la position dans un référentiel lié à un dispositif de capture d'images, et de l'inclinaison dans le plan sagittal de la tête du porteur, d'un référentiel lié à la tête du porteur, et un dispositif de capture d'images 90 pour capturer, dans un plan de capture d'images PCI, l'image du dispositif de repérage 20 monté sur la monture 10 en position de porté.

Le dispositif de capture d'images 90 peut être fixe ou mobile dans le référentiel terrestre. Dans le cas où il est mobile dans le référentiel terrestre, le dispositif de capture d'images 90 comporte des moyens de détermination de sa position et de son inclinaison dans le référentiel terrestre. Ces moyens de détermination peuvent par exemple comprendre un inclinomètre et/ou un accéléromètre.

Le dispositif de capture d'images 90 est relié à un système de traitement et de calcul 93 de l'image capturée.

Le plan de capture d'images PCI peut être orienté de différentes façons par rapport à la tête du porteur.

Dans l'exemple représenté sur la figure 3, ce plan de capture d'images PCI est frontal. Ce plan de capture d'images PCI frontal est défini comme étant perpendiculaire au plan sagittal PSAG et au plan de Francfort PF de la tête du porteur dans sa posture anatomique. Il s'agit du plan de la figure 2.

En variante, le plan de capture d'images du dispositif de capture d'images peut s'étendre selon un plan parallèle au plan sagittal de la tête du porteur, de sorte que l'image capturée est alors une image de profil de la tête du porteur.

Le plan de capture d'images peut également être orienté pour réaliser une capture d'image de trois quart de la tête du porteur.

Lesdits moyens de repérage peuvent comporter des marqueurs disposés sur la tête du porteur, des points remarquables du visage du porteur ou un dispositif rapporté sur la tête du porteur dont les formes sont connues. Ce dispositif peut être constitué par la monture de lunettes elle-même ou par un dispositif rapporté sur cette monture.

Après une étape d'étalonnage de ces moyens de repérage, la projection de ces moyens de repérage dans le plan de capture d'images, qui modifie la forme apparente desdits moyens de repérage, donne accès à la position, dans un référentiel lié à un dispositif de capture d'images, et à l'inclinaison, dans le plan sagittal de la tête du porteur, du référentiel lié à la tête du porteur.

Comme représenté sur la figure 3, on définit le référentiel associé à la monture, et donc lié à la tête du porteur, ayant un repère orthonormé (O,X,Y,Z) et matérialisé par le dispositif de repérage 20. Le centre O du repère de ce référentiel est le milieu du segment joignant les pinces de fixation 26, 27. L'axe X est horizontal et passe par les pinces 26, 27. L'axe Y est perpendiculaire au plan de Francfort, donc vertical lorsque la tête du porteur est dans sa posture anatomique.

Le plan OYZ correspond au plan sagittal PSAG de la tête du porteur ainsi qu'au plan de symétrie de la monture. L'axe OZ est parallèle à la médiatrice AO des yeux. Le plan OXZ est sensiblement parallèle au plan de Francfort PF et est donc horizontal lorsque la tête du porteur est dans sa posture anatomique (figure 3).

Le dispositif de repérage 20 est représenté sur la figure 4. Ce dispositif de repérage 20 et son utilisation sont décrits en détail dans le document FR2914173. Seuls les éléments principaux de ce dispositif de repérage 20 seront rappelés ici.

Le dispositif de repérage 20 comporte une ossature articulée comportant deux baguettes 23, 24 sensiblement rectilignes et sensiblement coplanaires, reliées entre elles par une charnière 29 présentant un axe d'articulation C1 sensiblement vertical dans la configuration du porté.

Chaque baguette 23, 24 est munie de pinces 25, 26, 27, 28 qui permettent de fixer, avec une capacité de pivotement, chaque baguette 23, 24 sur la partie supérieure sensiblement horizontale du cercle 11, 12 de la monture (figures 2 et 4) ou, lorsque les lunettes sont du type percées, de la lentille de présentation.

Chaque baguette 23, 24 est surmontée d'un élément de repérage horizontal 70, 80 qui se présente sous la forme d'une plaque triangulaire, d'une certaine épaisseur, dont une tranche présente une figure géométrique 71, 81 conçue de telle sorte que la configuration géométrique de cette figure géométrique 71, 81 projetée dans ledit plan de capture d'images PCI soit représentative de la composante horizontale de l'orientation de cet élément de repérage horizontal 70, 80 dans le référentiel lié à la tête du porteur.

La composante horizontale de l'orientation d'un élément dans le référentiel lié à la tête du porteur est définie par l'angle que fait la direction longitudinale de cet élément par rapport au plan OXY en projection dans le plan OXZ. De même, la composante verticale de l'orientation d'un élément est définie par l'angle que fait la direction longitudinale de cet élément par rapport au plan OXY en projection dans le plan OYZ.

Les deux éléments de repérage horizontal 70, 80 sont également reliés entre eux par un élément de repérage médian 190 qui est associé mécaniquement aux deux éléments de repérage horizontal 70, 80 de manière à rester constamment dans une position fixe par rapport à un plan vertical médian de symétrie de ces deux éléments 70, 80 sensiblement confondu avec le plan de symétrie de la monture (lui-même sensiblement confondu avec le plan sagittal PSAG de la tête du porteur).

Cet élément de repérage médian 190 porte une figure géométrique 193 constituée ici de bandes sombres espacées entre elles d'une distance connue.

L'image de ces bandes sombres, vue en projection dans le plan de capture d'image PCI par le dispositif de capture d'image 90, permet de calculer la distance séparant le dispositif de repérage 20 du dispositif de capture d'images 90 et de déterminer le facteur d'échelle de l'image capturée.

Le dispositif de repérage 20 comporte de plus un élément de repérage vertical 60 qui est également constitué par une plaque triangulaire d'une épaisseur donnée s'étendant dans un plan sensiblement perpendiculaire au plan moyen des deux éléments de repérage horizontal 70, 80 associés aux lentilles 100, 101. Cet élément de repérage vertical 60 est fixé sur la face supérieure de la barre de maintien 190 en son centre.

Il présente sur l'une de ses tranches, qui est destinée à être orientée vers le dispositif de capture d'images 90, une figure géométrique 61 constituée de motifs géométriques qui, comme précédemment, sont des bandes sombres séparées les unes des autres d'une distance connue et qui s'étendent selon la direction longitudinale de la tranche correspondante de l'élément de repérage 60.

Il en résulte qu'ici, chaque bande sombre est disposée sensiblement à l'horizontale dans la configuration du porté et que la direction longitudinale de la figure géométrique 61 est sensiblement verticale.

La hauteur des bandes sombres projetées dans le plan de capture d'images PCI permet de déterminer l'angle pantoscopique AMV de la monture 10 lorsque la tête du porteur est dans sa posture anatomique.

Les éléments de repérage horizontal 70, 80, médian 190 et vertical 60 du dispositif de repérage 20 permettent donc de repérer, dans un référentiel lié à la tête du porteur, l'inclinaison du dispositif de repérage 20 dans le plan sagittal, autour d'un centre de rotation de la tête du porteur, et de repérer, dans un référentiel lié au dispositif de capture d'images 90, la position du dispositif de repérage 20.

Le dispositif de capture d'images 90 consiste typiquement en une vidéo-caméra ou en un appareil de photographie numérique portatif ou monté sur support ou piètement.

L'axe optique de ce dispositif de capture d'images 90 s'étend de préférence dans un plan horizontal. La position et l'inclinaison de ce dispositif de capture d'images 90 restent, dans l'exemple développé ici, fixes pendant la capture d'une série d'image. En pratique, le déplacement de l'appareil de capture d'images 90 entre deux captures d'image successives est ici négligeable.

La position du dispositif de capture d'images 90 est telle que le visage du porteur est inclus dans son champ d'acquisition d'images.

En variante, le dispositif de capture d'images peut être incliné par rapport à l'horizon terrestre d'un angle quelconque connu ou mesuré par un inclinomètre par exemple. Une étape d'étalonnage est alors nécessaire.

On définit le référentiel lié au dispositif de capture d'images 90, muni d'un repère orthogonal (O1,X1,Y1,Z1) dans lequel l'axe O1Z1 est l'axe optique du dispositif de capture d'images 90.

Le système de traitement et de calcul 93 de l'image acquise comporte ici un microordinateur sur lequel est installé un logiciel de traitement et de calcul de l'image acquise. En variante, on peut prévoir que le système de traitement et de calcul soit un système autonome qui comporte, d'une part, un écran d'affichage pour communiquer les résultats obtenus et, d'autre part, une connectique pour permettre de communiquer ces résultats à d'autres appareils. On peut également prévoir dans le cas d'un système autonome de traitement que ce système soit intégré ou non au dispositif de capture d'images 90.

### Procédé

Le dispositif de détermination décrit ci-dessus permet de mettre en oeuvre le procédé suivant de détermination d'au moins un paramètre géométrico-postural d'implantation d'une monture de lunettes de correction visuelle sur le visage d'un porteur dans sa posture anatomique.

Comme représenté sur la figure 2, dans une première étape a) du procédé selon l'invention, l'opticien positionne la paire de lunettes de présentation surmontée du dispositif de repérage 20 sur le nez du porteur. Le porteur est en configuration assise ou debout.

Comme expliqué précédemment, le dispositif de repérage 20 autorise la détermination de la position dans le référentiel lié au dispositif de capture d'images 90 du référentiel lié à la tête du porteur.

Il donne également accès à l'écart d'inclinaison, dans le plan sagittal, entre deux captures d'image, du référentiel lié à la tête du porteur, dans le référentiel lié au dispositif de capture d'images 90.

Dans une deuxième étape b) du procédé, l'opticien demande au porteur d'effectuer un mouvement de hochement vertical de la tête dans son plan sagittal PSAG, en gardant le plan sagittal de sa tête vertical et sensiblement fixe latéralement, c'est-à-dire dans la direction perpendiculaire à ce plan sagittal. En pratique, le déplacement latéral global du plan sagittal de la tête pendant la capture d'une série d'images est maintenu inférieur à environ 5 degrés d'angle par rapport au dispositif de capture d'images 90.

En pratique, le porteur se regarde dans un miroir et effectue un hochement de la tête d'une amplitude de l'ordre de 20 degrés.

Pendant ce mouvement, le dispositif de capture d'images 90 capture une série d'au moins deux images de la tête du porteur, correspondant chacune à une inclinaison différente de la tête dans son plan sagittal, dans le référentiel lié au dispositif de capture d'images.

De préférence, le dispositif de capture d'images 90 enregistre une série d'images de la tête du porteur, correspondant chacune à une inclinaison différente de la tête du porteur dans son plan sagittal, et comportant au moins une image dans chaque intervalle de 1 degré compris dans l'amplitude du hochement de la tête.

Dans une autre étape c) du procédé, le système de traitement et de calcul 93 des images capturées traite ces images pour déterminer la position, dans le référentiel lié au dispositif de capture d'images, du centre de rotation Ai de la tête du porteur (figures 2 et 3).

Cette détermination est réalisée à partir des positions et des inclinaisons du référentiel lié à la tête du porteur fournies par les moyens de repérage et correspondant aux images capturées.

Dans le cas préféré d'une série comportant un grand nombre d'images capturées, à l'étape c), la détermination du centre de rotation est réalisée à partir des positions et des inclinaisons du repère lié à la tête du porteur correspondant à un groupe d'images capturées successivement de ladite série d'images.

Le système de traitement et de calcul 93 sélectionne de préférence des couples d'images successives de la série d'images capturées, et détermine, dans le référentiel lié au dispositif de capture d'images, les positions et les inclinaisons du référentiel lié à la tête du porteur fournies par le dispositif de repérage 20 et le système de traitement et de calcul 93 en déduit la position du centre de rotation Ai horizontal de la tête du porteur commun à chaque couple d'images capturées sélectionné.

Cette étape est schématisée sur la figure 6.

Pendant le hochement de la tête du porteur, le centre de rotation Ai de la tête du porteur se déplace dans le plan sagittal. Ce déplacement est supposé négligeable entre deux images capturées successivement par le dispositif de capture d'images. Le déplacement de ce centre de rotation dans le plan sagittal est supposé inférieur à 5 millimètres .

Le système de traitement et de calcul 93 calcule donc de préférence une pluralité de positions du centre de rotation Ai correspondant à différents groupes d'images capturées successivement de ladite série d'images, et correspondant par exemple à chaque couple d'images successives sélectionné.

Le système de traitement et de calcul 93 traite chaque couple d'images successives pour déterminer la position d'un point particulier Mi du référentiel lié à la tête du porteur dans le référentiel de capture d'images et l'écart d'inclinaison Bi du référentiel lié à la tête du porteur entre les deux images successives considérées dans le référentiel de capture d'images.

En l'espèce, l'écart d'inclinaison Bi du référentiel lié à la tête du porteur entre les deux images successives dans le référentiel de capture d'images est calculé à partir des informations fournies par le dispositif de repérage 20, par soustraction des angles d'inclinaison du dispositif de repérage 20 dans le référentiel de la tête du porteur fournis par ce dispositif de repérage 20 sur les deux images considérées.

A partir de la position des points Mi et de l'écart d'inclinaison Bi calculés pour deux images successives, le système de traitement et de calcul 93 en déduit la position du centre de rotation Ai correspondant au couple d'images successives considéré. Pour chaque couple d'images, ce centre de rotation Ai se trouve au sommet d'un triangle isocèle dont l'angle au sommet est égal à l'écart d'inclinaison Bi calculé, comme représenté sur la figure 6.

Dans une étape d) du procédé, le système de traitement et de calcul 93 identifie, sur chaque image capturée, l'image ICNi d'un premier point anatomique remarquable CNi du visage du porteur.

Puis dans une étape e), il détermine, pour chaque image capturée, l'angle d'inclinaison d'un plan anatomique de la tête du porteur passant par ledit centre de rotation Ai et par ledit point CNi, par rapport à un plan de référence lié au dispositif de capture d'images.

Pour cela, le système de traitement et de calcul 93 détermine, pour chaque couple d'images successives de ladite série d'images capturées, la position, dans le référentiel lié au dispositif de capture d'images, dudit point CNi du visage du porteur.

Cette étape est schématisée sur la figure 7.

Sur cette figure, on a superposé dans le plan de capture d'image PCI, deux images successives de la série d'images capturées, en indiquant la position de l'image ICNI du premier point anatomique remarquable CNi identifié sur chacune de ces images.

Le plan de capture d'images PCI étant dans cet exemple frontal, les positions des premiers points anatomiques remarquables CNi correspondants du visage du porteur se trouvent sur une droite horizontale passant par les images ICNI identifiées de ces points CNi et perpendiculaire au plan de capture d'images PCI.

En outre, ces deux points CNi se trouvent à égale distance du centre de rotation Ai calculé à l'étape c) correspondant au couple d'images considéré.

L'écart d'inclinaison Bi dans le plan sagittal autour du centre de rotation Ai de ces points CNi est également connu.

Le système de traitement et de calcul 93 résout le système d'équation correspondant pour en déduire, dans le référentiel lié au dispositif de capture d'images, la position du premier point anatomique remarquable CNi du visage du porteur dans la position de la tête du porteur correspondant à chaque image capturée.

A partir de la position de ce premier point anatomique remarquable CNi et de la position du centre de rotation Ai correspondant, le système de traitement et de calcul 93 détermine l'angle d'inclinaison Ei d'un plan anatomique de la tête du porteur passant par ledit centre de rotation Ai et par ledit point CNi remarquable, par rapport à un plan de référence lié au dispositif de capture d'images. Ce plan de référence est ici un plan horizontal. Le plan anatomique est ici perpendiculaire au plan sagittal de la tête du porteur.

Ici on peut également envisager que le premier point anatomique remarquable considéré corresponde à la projection dans le plan sagittal d'un point du visage du porteur et que le système de traitement et de calcul 93 calcule l'angle entre la droite passant par cette projection et le centre de rotation par rapport au plan de référence.

En variante, le système de traitement et de calcul 93 détermine la position de deux premiers points anatomiques remarquables et détermine l'angle d'inclinaison du plan anatomique de la tête du porteur passant par ledit centre de rotation et par ces deux premiers points anatomiques remarquables, par rapport à un plan de référence lié au dispositif de capture d'images.

Dans une étape f) dudit procédé, le système de traitement et de calcul 93 compare cet angle d'inclinaison avec une valeur prédéterminée.

Selon un premier mode de réalisation du procédé selon l'invention, à l'étape d), le système de traitement et de calcul 93 identifie, sur chaque image capturée, l'image d'au moins une commissure nasale CN ou temporale CT d'un oeil OD, OG du porteur (voir figure 2). Il identifie ici de préférence une commissure nasale CN d'un oeil du porteur.

Les commissures nasale, appelées endocanthions, et temporale, appelées ectocanthions, de chaque oeil sont définies comme les points extrêmes de chaque oeil, situés du côté du nez ou du côté de la tempe du porteur.

A l'étape e), le système de traitement et de calcul 93 détermine alors l'angle d'inclinaison Ei du plan anatomique de la tête du porteur passant par cette commissure CN, CT et par le centre de rotation Ai correspondant à chaque image, et perpendiculaire au plan sagittal. Ce plan anatomique est appelé plan neuro-orbital (PNO) et son angle d'inclinaison par rapport au plan de Francfort est une constante morphologique dont la valeur médiane pour une population d'individus donnée est connue statistiquement. Cette valeur médiane de l'angle d'inclinaison est égale à 7 degrés : ce plan neuro-orbital est situé environ 7 degrés au-dessus du plan de Francfort PF.

Ainsi, dans ce cas, la valeur prédéterminée à laquelle est comparé l'angle d'inclinaison Ei dudit plan anatomique passant par la commissure de l'oeil du porteur et le centre de rotation de sa tête est comprise entre 5 degrés et 9 degrés, et de préférence égale à 7 degrés.

On peut envisager également que ladite valeur prédéterminée soit ajustée en fonction du porteur, notamment en fonction de son âge ou de son ethnie.

On peut également envisager d'identifier l'image de deux, trois ou quatre commissures nasales CN et/ou temporales CT des yeux OD, OG du porteur et de déterminer l'angle d'inclinaison du plan anatomique moyen passant par ces deux, trois, ou quatre points.

Selon un deuxième mode de réalisation du procédé selon l'invention, à l'étape d), le système de traitement et de calcul 93 identifie sur chaque image capturée, la position du point inférieur du nez du porteur et, à l'étape e), détermine l'angle d'inclinaison du plan anatomique de la tête du porteur passant par ce point inférieur du nez et le centre de rotation correspondant à chaque image. Ce plan anatomique est appelé plan de Camper et son angle d'inclinaison par rapport au plan de Francfort est une constante morphologique dont la valeur médiane pour une population d'individus donnée est connue statistiquement. Cette valeur médiane de l'angle d'inclinaison est égal à -18 degrés : ce plan de Camper est situé environ 18 degrés en dessous du plan de Francfort PF.

Dans ce cas, la valeur prédéterminée à laquelle est comparé l'angle d'inclinaison dudit plan anatomique passant par le point inférieur du nez et par le centre de rotation de la tête du porteur est alors comprise entre -15 et -21 degrés, et de préférence égale à -18 degrés.

Enfin dans une étape g) du procédé, quel que soit le plan anatomique dont l'angle d'inclinaison est déterminé à l'étape e), le système de traitement et de calcul 93 détermine le paramètre géométrico-postural recherché en fonction du résultat de cette comparaison.

Pour cela, selon une première variante, à l'étape g),
- le système de traitement et de calcul 93 sélectionne l'image de ladite série d'images capturées pour laquelle l'écart entre l'angle d'inclinaison Ei déterminé à l'étape e) et ladite valeur prédéterminée est le plus petit,
- le système de traitement et de calcul 93 détermine le paramètre géométrico-postural recherché par traitement de cette image sélectionnée.

Ce traitement est décrit en détail dans le document FR2914173.

Eventuellement, on peut prévoir que le système de traitement et de calcul 93 corrige le paramètre géométrico-postural calculé en fonction de l'écart U entre l'angle d'inclinaison Ei déterminé à l'étape e) et ladite valeur prédéterminée. Cette étape de correction est schématisée sur la figure 8. Sur cette figure 8, le plan de Francfort PF de la tête du porteur est incliné de l'angle U par rapport au plan de référence PRef horizontal.

La hauteur apparente HApp de l'oeil droit OD, représenté sur la figure 8, déterminée par traitement de l'image correspondante est égale à la hauteur de cet oeil H recherchée dans la posture anatomique du porteur, c'est-à-dire lorsque le plan de Francfort PF est parallèle au plan de référence Pref horizontal, à laquelle s'ajoute une erreur CH.

Cette erreur CH est égale à la distance entre le centre de rotation de l'oeil CROD et la monture, distance qui est ici représentée par la distance entre le centre de rotation CROD et sa projection horizontale sur un plan moyen de la monture 10 correspondant au point T de la figure 8, multipliée par la tangente de l'angle U d'inclinaison du plan de Francfort par rapport au plan de référence.

On peut également prévoir que le système de traitement et de calcul 93 compare la valeur de l'angle d'inclinaison Ei calculé à l'étape e) de l'image sélectionnée avec deux valeurs seuil haute et basse égales à la valeur prédéterminée plus ou moins 0,5 degré. Si l'angle d'inclinaison Ei entre le plan anatomique et le plan de Francfort sur l'image sélectionnée est supérieur à la valeur seuil haute ou inférieur à la valeur seuil basse, le système de traitement et de calcul 93 indique à l'opticien qu'une autre capture d'une série d'images doit être réalisée.

Selon une deuxième variante, à l'étape g),
- le système de traitement et de calcul 93 sélectionne les deux images de ladite série d'images capturées pour lesquelles l'écart entre l'angle d'inclinaison Ei déterminé à l'étape e) et ladite valeur prédéterminée est le plus petit,
- le système de traitement et de calcul 93 calcule, à partir de ces deux images sélectionnées, une modélisation d'au moins une partie du visage du porteur correspondant à l'image qui serait effectivement capturée si la valeur de l'angle d'inclinaison Ei dudit plan anatomique était égale à ladite valeur prédéterminée.

Cette modélisation est par exemple déterminée par interpolation des deux images sélectionnées.

Le système de traitement et de calcul 9 détermine ensuite le paramètre géométrico-postural recherché par traitement de cette modélisation calculée du visage du porteur.

En variante de ce qui précède, la détermination de la position du centre de rotation de la tête du porteur peut être réalisée à partir d'une image sensiblement de profil de la tête du porteur.

On capture alors à l'étape b), au moins une image sensiblement de profil de la tête du porteur, c'est-à-dire capturée dans un plan sensiblement parallèle au plan sagittal de la tête du porteur et au moins une image de face de la tête du porteur, de préférence une série d'au moins deux images de la tête du porteur.

A l'étape c), la détermination de la position dudit centre de rotation de la tête du porteur comprend alors l'identification, sur ladite image sensiblement de profil capturée à l'étape b), d'un deuxième point anatomique remarquable, qui correspond au tragion de l'oreille du porteur.

L'identification de ce deuxième point anatomique remarquable peut être réalisée :
- soit par traitement de ladite image sensiblement de profil capturée à l'étape b) et reconnaissance sur cette image d'une forme associée à ce deuxième point anatomique remarquable,
- soit par l'affichage sur un écran de ladite image sensiblement de profil capturée à l'étape b) et la mise en oeuvre de moyen de pointage dudit deuxième point anatomique remarquable.

Le système de traitement et de calcul détermine alors la position dans le référentiel lié au dispositif de capture d'images du tragion identifié et en déduit la position, dans le référentiel lié au dispositif de capture d'images, du centre de rotation de la tête du porteur dans le plan sagittal. Le centre de rotation correspond en effet à la projection de ce tragion de la tête du porteur dans le plan sagittal.

Les autres étapes du procédé se déroulent comme exposées précédemment.

Quelle que soit la variante du procédé utilisée, le paramètre géométrico-postural recherché est ainsi déterminé soit sur une image du porteur très proche de sa posture anatomique, soit sur une modélisation du porteur dans sa posture anatomique. La précision du paramètre géométrico-postural ainsi déterminé est augmentée et la précision de la conception personnalisée des lentilles est améliorée, ce qui améliore le confort du porteur.

En particulier, on peut envisager tout type de moyens de repérage, notamment des marques de repère disposées sur la tête du porteur, par exemple des marques dessinées au feutre ou des pastilles collées sur la tête du porteur.

## Revendications

1. Procédé de détermination d'au moins un paramètre géométrico-postural (H, AMV) d'implantation d'une monture (10) de lunettes de correction visuelle sur le visage d'un porteur dans sa posture anatomique, selon lequel on réalise les étapes suivantes :
a) on définit, sur la tête du porteur et/ou sur la monture (10) de lunettes équipant le porteur, des moyens de repérage (20) pour repérer :
- la position d'un référentiel (O,X,Y,Z) lié à la tête du porteur dans un référentiel (O1,X1,Y1,Z1) lié à un dispositif de capture d'images (90) et
- l'inclinaison de ce référentiel lié à la tête du porteur dans le plan sagittal (PSAG) de la tête du porteur, autour d'un centre de rotation (Ai) de la tête du porteur,
le procédé **caractérisé en ce que** :
b) on capture une série d'au moins deux images de la tête dudit porteur par le dispositif de capture d'images (90)
c) on détermine au moins une position, dans le référentiel lié au dispositif de capture d'images (90), du centre de rotation (Ai) de la tête du porteur dans le plan sagittal (PSAG),
d) on identifie, sur chacune desdites au moins deux images capturées, l'image d'au moins un premier point anatomique (CNi) remarquable du visage du porteur,
e) on détermine, pour chacune desdites au moins deux images capturées, l'angle d'inclinaison (Ei) d'un plan anatomique (PNO) de la tête du porteur passant par ledit centre de rotation (Ai) et par ledit au moins un premier point anatomique (CNi) et perpendiculaire au plan sagittal (PSAG), par rapport à un plan de référence (PRef) connu dans le référentiel (O1,X1,Y1,Z1) lié au dispositif de capture d'images (90),
f) on compare cet angle d'inclinaison (Ei) avec une valeur prédéterminée,
g) on détermine le paramètre géométrico-postural (H, AMV) recherché en fonction du résultat de cette comparaison.

2. Procédé selon la revendication 1, selon lequel, à l'étape g),
- on sélectionne l'image de ladite série d'images capturées pour laquelle l'écart entre l'angle d'inclinaison (Ei) déterminé à l'étape e) et ladite valeur prédéterminée est le plus petit,
- on détermine le paramètre géométrico-postural (H, AMV) recherché par traitement de cette image sélectionnée.

3. Procédé selon la revendication 2, dans lequel, à l'étape g),
- on corrige le paramètre géométrico-postural (H, AMV) calculé en fonction de l'écart entre l'angle d'inclinaison (Ei) déterminé à l'étape e) pour l'image sélectionnée et ladite valeur prédéterminée.

4. Procédé selon la revendication 1, dans lequel, à l'étape g),
- on sélectionne les deux images de ladite série d'images capturées pour lesquelles l'écart entre l'angle d'inclinaison (Ei) déterminé à l'étape e) et ladite valeur prédéterminée est le plus petit,
- on calcule, à partir de ces deux images sélectionnées, une modélisation d'au moins une partie du visage du porteur correspondant à l'image qui serait effectivement capturée si la valeur de l'angle d'inclinaison (Ei) dudit plan anatomique (PNO) était égale à ladite valeur prédéterminée,
- on détermine, à partir de cette modélisation le paramètre géométrico-postural (H, AMV) recherché.

5. Procédé selon l'une des revendications précédentes, dans lequel, à l'étape a), lesdits moyens de repérage (20) comportent un dispositif de repérage (20) monté directement sur la tête du porteur ou sur la monture (10) de lunettes de ce porteur.

6. Procédé selon l'une des revendications précédentes, dans lequel, à l'étape a), lesdits moyens de repérage comportent des marques de repère disposées sur la tête du porteur.

7. Procédé selon l'une des revendications précédentes, dans lequel, à l'étape d), on identifie, sur chacune desdites au moins deux images capturées, l'image d'au moins une commissure nasale (CN) ou temporale (CT) d'un oeil (OD, OG) du porteur et, à l'étape e), on détermine l'angle d'inclinaison (Ei) du plan anatomique (PNO) de la tête du porteur passant par cette commissure (CN, CT) et par le centre de rotation correspondant à cette image.

8. Procédé selon l'une des revendications 1 à 6, dans lequel, à l'étape d), on identifie sur chacune desdites au moins deux images capturées, la position du point le plus bas du nez du porteur, appelé point sous-nasal, et, à l'étape e), on détermine l'angle d'inclinaison du plan anatomique de la tête du porteur passant par ce point sous-nasal et le centre de rotation correspondant à cette image.

9. Procédé selon l'une des revendications précédentes, dans lequel, à l'étape g), le paramètre géométrico-postural (H, AMV) déterminé comprend l'une au moins des grandeurs suivantes :
- l'angle pantoscopique (AMV) de la monture (10) équipant le porteur,
- la hauteur (H) des centres de rotation (CROG, CROD) des yeux du porteur par rapport au bord inférieur de cette monture (10).

10. Procédé selon l'une des revendications précédentes, dans lequel, à l'étape b), le porteur effectuant un mouvement de hochement vertical de la tête sensiblement dans le plan sagittal, on capture une série d'au moins deux images de la tête du porteur par le dispositif de capture d'images (90), pour au moins deux inclinaisons différentes de la tête.

11. Procédé selon la revendication 10, dans lequel, à l'étape c), on détermine la position du centre de rotation (Ai) à partir des positions et des inclinaisons du repère lié à la tête du porteur fournies par les moyens de repérage (20) et correspondant à au moins deux desdites images capturées.

12. Procédé selon la revendication 11, dans lequel, à l'étape c), la détermination du centre de rotation (Ai) est réalisée à partir des positions et des inclinaisons du repère lié à la tête du porteur correspondant à un groupe d'images capturées successivement de ladite série d'images.

13. Procédé selon la revendication 12, dans lequel, à l'étape c), on détermine une pluralité de positions du centre de rotation (Ai) correspondant à différents groupes d'images capturées successivement de ladite série d'images.

14. Procédé selon l'une des revendications 10 à 13, dans lequel, à l'étape b), toutes les images capturées du porteur sont des images sensiblement frontales du porteur, c'est-à-dire capturées dans un plan sensiblement perpendiculaire au plan sagittal (PSAG) de la tête du porteur.

15. Procédé selon l'une des revendications 1 à 13, dans lequel :
- à l'étape b), on capture au moins une image sensiblement de profil de la tête du porteur, c'est-à-dire capturée dans un plan sensiblement parallèle au plan sagittal (PSAG) de la tête du porteur.
- à l'étape c), la détermination de la position dudit centre de rotation (Ai) de la tête du porteur comprend l'identification, sur ladite image sensiblement de profil capturée à l'étape b), d'un deuxième point anatomique remarquable (TR).

16. Procédé selon la revendication 15, dans lequel, à l'étape c), l'identification dudit deuxième point anatomique remarquable (TR) est réalisée :
- par traitement de ladite image sensiblement de profil capturée à l'étape b) et reconnaissance sur cette image d'une forme associée à ce deuxième point anatomique remarquable (TR) ou
- par l'affichage sur un écran de ladite image sensiblement de profil capturée à l'étape b) et la mise en oeuvre de moyen de pointage dudit deuxième point anatomique remarquable (TR).

## Claims

1. A method of determining at least one geometrical-and-postural parameter (H, AMV) for positioning a frame (10) of vision-correcting eyeglasses on the face of a wearer in the anatomical posture, wherein the following steps are performed:
a) defining position-identification means (20) on the wearer's head and/or on the eyeglass frame (10) worn by the wearer in order to identify:
· the position of a frame of reference (O,X,Y,Z) associated with the wearer's head in a frame of reference (O1,X1,Y1,Z1) associated with an image-capture device (90); and
. the angle of inclination of said frame of reference associated with the wearer's head in the sagittal plane (PSAG) of the wearer's head about a center of rotation (Ai) of the wearer's head;
the method being **characterized in that** the following steps are performed:
b) capturing a series of at least two images of the head of said wearer by means of the image-capture device (90);
c) determining in the frame of reference associated with the image-capture device (90), at least one position of the center of rotation (Ai) of the wearer's head in the sagittal plane (PSAG);
d) in each of said at least two captured images, identifying the image of at least one first remarkable anatomical point (CNi) of the wearer's face;
e) for each of said at least two captured images, determining the angle of inclination (Ei) relative to a reference plane (PRef) known in the frame of reference (O1,X1,Y1,Z1) associated with the image-capture device (90) of an anatomical plane (PNO) of the wearer's head that is perpendicular to the sagittal plane (PSAG) and that contains said center of rotation (Ai) and said at least one first anatomical point (CNi);
f) comparing said angle of inclination (Ei) with a predetermined value; and
g) determining the looked-for geometrical-and-postural parameter (H, AMV) as a function of the result of said comparison.

2. A method according to claim 1, wherein, in step g):
. the image in said series of captured images is selected for which the difference between the angle of inclination (Ei) as determined in step e) and said predetermined value is the smallest; and
. the looked-for geometrical-and-postural parameter (H, AMV) is determined by processing said selected image.

3. A method according to claim 2, wherein, in step g):
. the calculated geometrical-and-postural parameter (H, AMV) is corrected as a function of the difference between the angle of inclination (Ei) as determined in step e) for the selected image and said predetermined value.

4. A method according to claim 1, wherein, in step g):
. the two images of said series of captured images are selected so that the difference between the angle of inclination (Ei) determined in step e) and said predetermined value is the smallest;
. on the basis of said two selected images, a model is calculated of at least a portion of the wearer's face corresponding to the image that would have been captured if the value of the angle of inclination (Ei) of said anatomical plane (PNO) was equal to said predetermined value; and
. on the basis of said model, the looked-for geometrical-and-postural parameter (H, AMV) is determined.

5. A method according to any preceding claim, wherein, in step a), said position-identification means (20) comprise a position-identification device (20) mounted directly on the wearer's head or on the frame (10) of the wearer's eyeglasses.

6. A method according to any preceding claim, wherein, in step a), said position-identification means comprise identifying marks placed on the wearer's head.

7. A method according to any preceding claim, wherein, in step d), on each of said at least two captured images, the image is identified of at least one nasal or temporal corner (CN, CT) of an eye (OD, OG) of the wearer, and in step e), the angle of inclination (Ei) is determined of the anatomical plane (PNO) of the wearer's head containing said corner (CN, CT) and the center of rotation corresponding to said image.

8. A method according to any one of claims 1 to 6, wherein, in step d), in each of said at least two captured images, the position of the lowest point of the wearer's nose, referred to as the sub-nasal point, is identified, and in step e), the angle of inclination is determined of the anatomical plane of the wearer's head containing said sub-nasal point and the center of rotation corresponding to said image.

9. A method according to any preceding claim, wherein, in step g), the geometrical-and-postural parameter (H, AMV) that is determined comprises at least one of the following magnitudes:
. the pantoscopic angle (AMV) of the frame (10) worn by the wearer; and
. the heights (H) of the centers of rotation (CROG, CROD) of the wearer's eyes relative to the bottom edge of the frame (10).

10. A method according to any preceding claim, wherein, in step b), the wearer performs a vertical nodding movement of the head substantially in the sagittal plane, and a series of at least two images of the wearer's head is captured by the image-capture device (90) for at least two different angles of inclination of the head.

11. A method according to claim 10, wherein, in step c), the position of the center of rotation (Ai) is determined from the positions and the angles of inclination of the frame of reference associated with the wearer's head as provided by the position-identification means (20) and corresponding to at least two of said captured images.

12. A method according to claim 11, wherein, in step c), the position of the center of rotation (Ai) is determined from the positions and the angles of inclination of the frame of reference associated with the wearer's head and corresponding to a group of images that were captured in succession in said series of images.

13. A method according to claim 12, wherein, in step c), a plurality of positions are determined for the center of rotation (Ai) corresponding to different groups of successively-captured images in said series of images.

14. A method according to any one of claims 10 to 13, wherein, in step b), all of the captured images of the wearer are substantially frontal images of the wearer, i.e. images captured in a plane that is substantially perpendicular to the sagittal plane (PSAG) of the wearer's head.

15. A method according to any one of claims 1 to 13, wherein:
. in step b), at least one substantially profile image of the wearer's head is captured, i.e. an image is captured in a plane substantially parallel to the sagittal plane (PSAG) of the wearer's head; and
. in step c), the determination of the position of said center of rotation (Ai) of the wearer's head comprises identifying a second remarkable anatomical point (TR), in said substantially profile image captured in step b).

16. A method according to claim 15, wherein, in step c), said second remarkable anatomical point (TR) is identified:
· by processing said substantially profile image captured in step b) and by recognizing in said image a shape associated with said second remarkable anatomical point (TR); or
. by displaying said substantially profile image captured in step b) on a screen and by using pointer means to point to said second remarkable anatomical point (TR).

## Patentansprüche

1. Verfahren zur Ermittlung von mindestens einem geometrisch-posturalen Parameter (H, AMV) für den Sitz einer Brillenfassung (10) mit Brillengläsern zur Korrektur von Sehfehlern im Gesicht eines Brillenträgers in seiner anatomischen Haltung, bei dem folgende Schritte ausgeführt werden:
a) man definiert auf dem Kopf des Brillenträgers und/oder auf der Brillenfassung (10) des Brillenträgers Kennzeichnungsmittel (20) zum Kennzeichnen von:
- der Position eines Bezugssystems (O, X, Y, Z) in Bezug zum Kopf des Brillenträgers in einem Bezugssystem (01, X1, Y1, Z1) in Verbindung mit einer Bildaufnahmevorrichtung (90) und
- der Neigung dieses Bezugssystems in Bezug zum Kopf des Brillenträgers in der sagittalen Ebene (PSAG) des Kopfes des Brillenträgers um einen Rotationsmittelpunkt (Ai) des Kopfes des Brillenträgers,
wobei das Verfahren **dadurch gekennzeichnet ist, dass**:
b) man mit der Bildaufnahmevorrichtung (90) eine Reihe von mindestens zwei Bildern des Kopfes des Brillenträgers aufnimmt
c) man in einem Bezugssystem in Verbindung mit der Bildaufnahmevorrichtung (90) mindestens eine Position des Rotationsmittelpunktes (Ai) des Kopfes des Brillenträgers in der sagittalen Ebene (PSAG) ermittelt,
d) man auf jedem dieser mindestens zwei aufgenommenen Bilder das Bild von mindestens einem ersten anatomischen Kennpunkt (CNi) des Gesichts des Brillenträgers identifiziert,
e) man für jedes dieser mindestens zwei aufgenommenen Bilder in Bezug zu einer im Bezugssystem (01, X1, Y1, Z1) für die Bildaufnahmevorrichtung (90) bekannten Referenzebene den Neigungswinkel (Ei) einer anatomischen Ebene (PNO) des Kopfes des Brillenträgers ermittelt, der durch den Rotationsmittelpunkt (Ai) und durch den mindestens einen ersten anatomischen Punkt (CNi) führt und senkrecht zur sagittalen Ebene (PSAG) ist,
f) man diesen Neigungswinkel (Ei) mit einem vorbestimmten Wert vergleicht,
g) man den gesuchten geometrisch-posturalen Parameter (H, AMV) in Abhängigkeit des Ergebnisses dieses Vergleichs ermittelt.

2. Verfahren nach Anspruch 1, bei dem man im Schritt g)
- das Bild in der Reihe der aufgenommenen Bilder auswählt, bei dem die Abweichung zwischen dem im Schritt e) ermittelten Neigungswinkel (E) und dem vorbestimmten Wert am kleinsten ist,
- den gesuchten geometrisch-posturalen Parameter (H, AMV) durch Bearbeitung dieses ausgewählten Bildes ermittelt.

3. Verfahren nach Anspruch 2, bei dem man im Schritt g)
- den berechneten geometrisch-posturalen Parameter (H, AMV) in Abhängigkeit der im Schritt e) ermittelten Abweichung zwischen dem Neigungswinkel (Ei) für das gewählte Bild und dem vorbestimmten Wert korrigiert.

4. Verfahren nach Anspruch 1, bei dem man im Schritt g)
- die zwei Bilder der Reihe von aufgenommenen Bildern auswählt, bei denen die Abweichung zwischen dem im Schritt e) ermittelten Neigungswinkel (Ei) und dem vorbestimmten Wert am kleinsten ist,
- anhand dieser zwei ausgewählten Bilder eine Modellbildung von mindestens einem Teil des Gesichtes des Brillenträgers berechnet, das dem Bild entspricht, das tatsächlich aufgenommen werden würde, wenn der Wert des Neigungswinkels (Ei) der anatomischen Ebene (PNO) dem vorbestimmten Wert entspricht,
- anhand dieser Modellbildung den gesuchten geometrisch-posturalen Parameter (H, AMV) ermittelt.

5. Verfahren nach einem der vorausgehenden Ansprüche, bei dem die Kennzeichnungsmittel (20) im Schritt a) eine direkt auf den Kopf des Brillenträgers oder auf die Brillenfassung (10) dieses Brillenträgers montierte Kennzeichnungsvorrichtung (20) umfassen.

6. Verfahren nach einem der vorausgehenden Ansprüche, bei dem die Kennzeichnungsmittel im Schritt a) auf dem Kopf des Brillenträgers angeordnete Kennzeichnungspunkte umfassen.

7. Verfahren nach einem der vorausgehenden Ansprüche, bei dem man im Schritt d) auf jedem der mindestens zwei aufgenommenen Bilder das Bild von mindestens einem Nasen-(CN) oder Schläfenwinkel (CT) eines Auges (OD, OG) des Brillenträgers identifiziert und man im Schritt e) den Neigungswinkel (Ei) der anatomischen Ebene (PNO) des Kopfes des Brillenträgers ermittelt, der durch diesen Winkel (CN, CT) und durch den diesem Bild entsprechenden Rotationsmittelpunkt führt.

8. Verfahren nach einem der Ansprüche 1 bis 6, bei dem man im Schritt d) auf jedem dieser mindestens zwei aufgenommenen Bilder die Position des untersten Punktes der Nase des Brillenträgers (unterer Nasenpunkt genannt) identifiziert und man im Schritt e) den Neigungswinkel der anatomischen Ebene des Kopfes des Brillenträgers ermittelt, der durch diesen unteren Nasenpunkt und durch den diesem Bild entsprechenden Rotationsmittelpunkt führt.

9. Verfahren nach einem der vorausgehenden Ansprüche, bei dem der im Schritt g) ermittelte geometrisch-posturale Parameter (H, AMV) mindestens eine der folgenden Größen umfasst:
- den pantoskopischen Winkel (AMV) der Brillenfassung (10) des Brillenträgers,
- die Höhe (H) der Rotationsmittelpunkte (CROG, CROD) der Augen des Brillenträgers in Bezug zum unteren Rand dieser Brillenfassung (10).

10. Verfahren nach einem der vorausgehenden Ansprüche, bei dem man im Schritt b), wenn der Brillenträger mit dem Kopf, der sich in leichter sagittaler Ebene befindet, nickt, eine Reihe von mindestens zwei Bildern des Kopfes des Brillenträgers mit der Bildaufnahmevorrichtung (90) für mindestens zwei verschiedene Neigungen des Kopfes aufnimmt.

11. Verfahren nach Anspruch 10, bei dem man im Schritt c) anhand der von den Kennzeichnungsmitteln (20) und mindestens zweien der aufgenommenen Bilder entsprechend bereitgestellten Positionen und der Neigungen der Kennzeichnung in Bezug zum Kopf des Brillenträgers die Position des Rotationsmittelpunktes (Ai) ermittelt.

12. Verfahren nach Anspruch 11, bei dem der Rotationsmittelpunkt (Ai) im Schritt c) anhand von Positionen und Neigungen der Kennzeichnung in Bezug zum Kopf des Brillenträgers entsprechend einer nacheinander aufgenommenen Bildergruppe der Bilderreihe ermittelt wird.

13. Verfahren nach Anspruch 12, bei dem man im Schritt c) eine Vielzahl von Positionen des Rotationsmittelpunktes (Ai) entsprechend verschiedener Gruppen von nacheinander aufgenommenen Bildern der Reihe von Bildern ermittelt.

14. Verfahren nach einem der Ansprüche 10 bis 13, bei dem alle in Schritt b) aufgenommenen Bilder des Brillenträgers im Wesentlichen frontale Aufnahmen des Brillenträgers sind, das heißt Bilder, die in einer annähernd senkrechten Ebene zur sagittalen Ebene (PSAG) des Kopfes des Brillenträgers aufgenommen werden.

15. Verfahren nach einem der Ansprüche 1 bis 13, bei dem:
- man im Schritt b) mindestens eine annähernde Profilaufnahme des Kopfes des Brillenträgers macht, das heißt ein Bild, das in einer annähernd parallelen Ebene zur sagittalen Ebene (PSAG) des Kopfes des Brillenträgers aufgenommen wird.
- im Schritt c) die Bestimmung der Position des Rotationsmittelpunktes (Ai) des Kopfes des Brillenträgers die Identifikation eines zweiten anatomischen Kennpunktes (TR) auf dem Bild der in Schritt b) gemachten annähernden Profilaufnahme umfasst.

16. Verfahren nach Anspruch 15, bei dem im Schritt c) die Identifikation des zweiten anatomischen Kennpunktes (TR) erfolgt durch:
- Bearbeiten der annähernden Profilaufnahme aus Schritt b) und Erkennen einer diesem zweiten anatomischen Kennpunkt (TR) assoziierten Form auf diesem Bild oder
- Anzeige der annähernden Profilaufnahme aus Schritt b) auf einem Bildschirm und Einsetzen von Visiermitteln für den zweiten anatomischen Kennpunkt (TR).
